# EUROPEAN PATENT SPECIFICATION

(11) **EP 0 565 198 B1**
(45) Date of publication and mention of the grant of the patent: **03.09.1997**
(21) Application number: 93201016.8
(22) Date of filing: 06.04.1993
(51) Int. Cl.: C07C 2/58

(54) **Process for upgrading a paraffinic feedstock**
Verfahren zur Verbesserung einer paraffinischen Beschichtung
Procédé pour la valorisation d'une charge paraffinique

(30) Priority: 08.04.1992 EP 92201015
(43) Date of publication of application: 13.10.1993
(73) Proprietor: SHELL INTERNATIONALE RESEARCH MAATSCHAPPIJ B.V., 2596 HR Den Haag (NL)
(72) Inventor: Peferoen, Danny Gaston René, NL-1031 CM Amsterdam (NL); Gilson, Jean-Pierre, NL-1031 CM Amsterdam (NL); Sie, Swan Tiong, NL-1031 CM Amsterdam (NL); De Jong, Krijn Pieter, NL-1031 CM Amsterdam (NL); Stork, Willem Hartman Jurriaan, NL-1031 CM Amsterdam (NL); Mesters, Carolus Matthias Anna Maria, NL-1031 CM Amsterdam (NL)

(56) References cited:
- FR-A- 2 631 956
- US-A- 5 073 665

## Description

The present invention relates to a process for upgrading a paraffinic feedstock. More specifically the invention relates to a process for alkylation of a paraffinic feedstock by the condensation of paraffins with olefins.

The production of highly branched hydrocarbons such as trimethylpentanes is important by virtue of their use as gasoline blending components of high octane number. Traditional production of highly branched hydrocarbons is by condensation of isobutane with light olefins, usually butenes but sometimes mixtures of propene, butenes and possibly pentenes using large quantities of conventional strong liquid acid catalysts, such as hydrofluoric or sulphuric acids. An emulsion of immiscible acid and hydrocarbon is agitated to emulsify the catalyst and reactant and refrigerated to control the highly exothermic reaction. By fine control of a complex interrelation of process variables high quality alkylate production may be maintained. The acid is recycled after use. It is desirable to use a process which is less hazardous and environmentally more acceptable.

Processes have been proposed to overcome these problems by using solid acids as catalysts. However paraffin olefin condensation yields both desired alkylate and undesired oligomerisation product. When catalyzing alkylation with solid acids it has been found that the selectivity for alkylate over oligomerisation product is less than that obtained with liquid acids. Moreover oligomerisation products are thought to cause the observed progressive deactivation of the catalyst. Regeneration techniques are known for removing hydrocarbonaceous deposits from solid catalysts and restoring catalyst activity. Nevertheless using known regeneration techniques for example raising the catalyst to elevated temperatures and oxidising the deposits, the alkylation must be interrupted and reactor conditions altered causing lost production time.

In US Patent No. 3,706,814 a process is disclosed for alkylation of isoparaffins using acidic zeolite catalysts and supplying to the reactor paraffin and olefin in a ratio of between 15 and 30, the concentration of unreacted olefin in the reactor maintained at less than 12 mole percent. Specifically the process is operated in a continuous stirred reactor using a zeolite Y catalyst. However from the results of the experiments severe deactivation is seen to occur already at low catalyst ages. In the process of French patent No. 2,631,956 isobutane and butene are reacted over zeolite-beta catalyst in an upflow fixed bed reactor. Product analysis at 1 hour and 4 hours time on stream shows a decrease in the percentage olefin conversion level in the reactor with time on stream. There is a need for an alkylation process which selectively yields highly branched alkylate at an acceptable rate for prolonged periods on stream. It has now surprisingly been discovered that by operation of a solid acid catalyzed alkylation process at a high extent of external circulation of liquid reactor content the rate of catalyst deactivation may be significantly decreased. Furthermore it has surprisingly been discovered that by such operation particularly beneficial selectivity for highly branched paraffins is achieved for the duration of the catalyst activity.

By the use of this process it has been discovered that the alkylation activity of a zeolite beta-type catalyst may be substantially improved.

Accordingly the present invention provides a process for upgrading a paraffinic feedstock comprising:
a) supplying the feedstock and an olefins-containing stream at a paraffin to olefin ratio greater than 2 v/v to a reactor containing a zeolite-beta catalyst and
b) removing the upgraded product wherein the process is operated at an olefin conversion of at least 90 mol% in an external
circulation reactor having an extent of external circulation of liquid reactor content expressed as$\frac{\text{Øvc + Øv}}{\text{Øv}} \text{of greater than 2}$ wherein Øv is the volumetric flow rate of feedstock and olefins-containing stream, and Øvc is the volumetric external circulation flow rate.

Reference herein to paraffin to olefin ratios are made as ratios by volume unless otherwise stated. By the term paraffin to olefin ratio is meant the quantity of feedstock paraffin per unit quantity of olefin introduced into the reactor not including paraffinic content of externally circulated liquid reactor contents.

Reference herein by the term alkylate is to a mixed condensation product of paraffin with olefin(s), and by the term oligomerisation product is to a condensation product of a plurality of olefin molecules. Alkylate is characterised by a higher motor octane number (MON) than oligomerisation product. Typically alkylate comprising highly branched paraffins with 5 to 12 carbon atoms has a MON of 86 or above, for example in the range 90 to 94 whilst a corresponding oligomerisation product may comprise a mixture of paraffinic or olefinic hydrocarbons typically of MON of less than 85, for example in the range 80 to 82.

External circulation of liquid reactor content as herein claimed and described refers to a portion of liquid reactor content isolated from the remainder of the liquid reactor content and transported via transport means such as for example conduit means to re-enter the reactor. An external circulation means may be external to the reactor but wholly or partially surrounded by the reactor for substantially all or part of its length, for example as a conduit means across the reactor comprised of a reactor inner wall or integral with the reactor outer wall. A high extent of external circulation of liquid reactor content is suitably attained in known reactor types such as for example solid phase fixed bed reactors or liquid phase continuous stirred tank reactors operated with external circulation along part or all of the length of the reactor and optionally operated with feed cross-flow. In the process of the invention the liquid reactor contents consists essentially of upgraded product, that is unreacted paraffin and alkylate. It is possible to externally circulate a part of the upgraded product since the alkylate comprised therein appears to be essentially stable under the applied process conditions to further reaction in the presence of catalyst. The remainder of the upgraded product is fed to a separation unit such as a distillation column for separation of alkylate from unreacted paraffin. Unreacted paraffin may be reused as feedstock paraffin and is suitably fed back to the feedstock inlet by known means. A particular advantage of the invention is the use of upgraded product for external circulation without the need for an intermediate alkylate separation stage. Suitable means may be used to regulate the rates of introduction of paraffin, olefin, the rate of removal of upgraded product and the rate of external circulation. Suitable means are used to divide off a portion of upgraded product for circulation. Such means preferably are of high throughput capacity and are physical separation means. Therefore conveniently upgraded product for external circulation may be drawn from the reactor effluent in which case a T-junction with regulated flow may be included in the reactor outlet.

The process may be carried out in a single reactor but is advantageously carried out in a plurality of reactors intercommunicating in series as a cascade. A cascade of reactors may be housed in a single unit or in separated units. Increased capacity may be achieved by operation of a cascade of reactors in parallel. The conditions according to the process of the invention may be attained in other known reactor types.

Volumetric flow rates are determined in the reactor effluent and external recycle by known techniques for example: by use of a rotameter, or by establishing the capacity of the recycle pump and the setting of the regulating flow valve to measure flow rate; by use of an anemometer or pitot tube for measurement of differential pressure; or by use of a mass flow meter together with density determination. Suitable apparatus and techniques are disclosed in "Chemical Engineer's Handbook" Perry and Chilton, McGraw-Hill, 1973, 5th edn. at 5-8 to 5-14.

Olefin conversion is defined as the ratio of olefin consumed in the reactor to the olefin introduced. At a high level of olefin conversion the desired product alkylate is obtained. High olefin conversion levels may be achieved by use of appropriate olefin space velocities suitably in excess of 0.01 kg/kg.h and high activity catalysts together with appropriate reactor temperature, composition and rate of introduction of paraffin and olefin and removal of upgraded product during the reaction. The level of olefin conversion is conveniently measured by gas chromatographic techniques in the effluent of the reactor.

The process is advantageously operated at an olefin space velocity at which at least 90 mol% olefin conversion is obtained. Olefin space velocity is defined as the weight of olefin introduced into the reactor per unit time per unit weight of catalyst in the reactor. A suitable olefin space velocity may lie in the range 0.01 to 10 kg/kg.h. Nevertheless operating conditions may be selected as required whereby olefin space velocity is increased above the optimum for ease of operation at sacrifice of some catalyst life. Catalyst life may be defined as the catalyst age at which deactivation is substantially complete. Catalyst age is defined as weight olefin supplied to the reactor per unit catalyst weight in the reactor, this being calculated as the product of olefin space velocity and time on stream. It has been found that the process of the invention may be operated at high catalyst ages before deactivation of the catalyst takes place. Operation beyond a catalyst life of 7 kg/kg and up to lives of the order of 15 kg/kg has been attained.

As a further advantage of the invention, operation at high olefin conversion levels in a reactor with external circulation according to the invention results in high alkylate yields selective to highly branched alkylates throughout the progressive deactivation of the catalyst. Only at full deactivation has olefin breakthrough been observed. Thus it may be economical to operate the process for the full catalyst life.

Excellent results are achieved with the process of the invention when operating in a single reactor. In a preferred embodiment, the process of the invention is operated in a cascade of reactors with split feed to each reactor whereby lower volumetric external circulation flow rates may be used. Suitably up to ten reactors may be used, preferably up to eight and most preferably five. Feed supply to each operating bed of a multiple fixed bed downflow reactor for example suitably comprises individual feed lines supplied from a central feed line.

A preferred operation of the process of the invention is in the preparation of highly branched paraffins containing 5 to 12 carbon atoms, preferably containing 5,6,7,8,9 or 12 carbon atoms, most preferably trimethyl-butane, -pentanes or -hexanes. The process may be applied in the upgrading of a paraffinic feedstock comprising iso-paraffins having from 4 to 8 carbon atoms as desired, suitably of a feedstock comprising isobutane, 2-methylbutane, 2,3-dimethylbutane, 3-methylhexane or 2,4-dimethylhexane, most preferably a paraffinic feedstock comprising iso-paraffins having 4 or 5 carbon atoms. Suitable feedstocks for the process of the invention include iso-paraffin containing fractions of oil conversion products such as naphtha fractions, and refined iso-paraffin feedstocks such as refined iso-butane. As hereinabove mentioned, it is convenient to isolate the iso-paraffin content of the effluent stream of liquid reactor products, for re-use as feedstock.

The olefins-containing stream is suitably in the form of a lower olefin containing hydrocarbon which may optionally contain additional non-olefinic hydrocarbons. Suitably the olefins-containing stream comprises ethene, propene, iso- or 1- or 2-(cis or trans) butene or pentene optionally diluted for example with propane, iso-butane, n-butane or pentanes. The process of the invention may suitably be operated downstream of a fluid catalytic cracking unit, an MTBE etherification unit or an olefin isomerization unit.

Preferably the reactor is started up in such a manner as to control initial contact of the catalyst with olefin feed. Suitably the reactor is first charged with paraffinic feedstock. An external circulation of liquid reactor contents is fed back to the fresh feed stream and an olefins-containing stream is subsequently continuously and quantitatively charged with the paraffinic feedstock at an olefin space velocity such that conversion is maintained in excess of 90 mol%. The feedstock and olefins stream may advantageously be charged via a plurality of inlet ports for example in cross-flow configuration. The use of nozzles to charge the reactor may advantageously provide improved dispersion of charge at the site of introduction. Suitable nozzles are those known for use in alkylation reactions using hydrogen fluoride as catalyst and nozzles known for dispersion of feed in stirred tank reactors and fixed catalyst beds. A cascade of reactors for example a multiple bed reactor may be operated by withdrawal of contents below a reactor for external circulation to previous reactors.

The alkylation reaction is preferably carried out at a temperature less than 150 °C, more preferably between 60 and 120 °C. Reaction pressure may be between 1 and 40 atmospheres. Suitably the pressure is above the vapour pressure of the reactor contents so as to maintain the reaction in the liquid phase.

Preferred paraffin to olefin ratio is in excess of 5 v/v, more preferably in the range of 10 to 30 v/v, most preferably in the range of 15 to 30 v/v. A low paraffin to olefin ratio suitably within the range 2 to 10 v/v favours the formation of higher alkylates, such as isoparaffins containing 12 carbon atoms.

Preferred olefin conversion substantially throughout the reactor is at least 95 mol%, more preferably at least 98 mol%, more preferably at least 99 mol%, such as for example 99.5, 99.8 or 99.9 mol%. Most preferably olefin conversion is substantially complete, i.e substantially 100%.

Preferably a reactor may be employed having an extent of external circulation of liquid reactor contents greater than 50, more preferably greater than 60 for example in the range 75 to 150 and most preferably in the range 80 to 120.

Preferred operating ranges of olefin space velocity include from 0.01 to 10 kg/kg.h, preferably from 0.01 to 5, more preferably from 0.01 to 0.5 kg/kg.h most preferably from 0.05 to 0.10 kg/kg.h.

The process may advantageously be operated by application of Advanced Process Control and optionally On-line Optimisation techniques using respectively multivariable dynamic and static models for the constraint control of selected process parameters. In a preferred operation of the process of the invention the alkylate obtained during operation is monitored and quality data together with the relevant operational process data are fed to an advanced process control system. Real time solutions of the advanced process controller are automatically fed back to a process implementation system and implemented in order to ensure the process parameters remain within operating constraints. Beneficial results may be obtained by maintaining process parameters such as volumetric flow rates, temperature and olefin space velocity within predetermined constraints.

A zeolite-beta catalyst used in the process of the invention may be any catalyst which conforms to the structural classification of a zeolite-beta, suitably as in Newman, Treacy et al., Proc. R. Soc. London Ser. A 420, 375, (1988).

The zeolitic catalyst is suitably present on a support. For example the catalyst suitably further comprises a refractory oxide that serves as binder material such as alumina, silica-alumina, magnesia, titania, zirconia and mixtures thereof. Alumina is specially preferred. The weight ratio of refractory oxide and zeolite suitably ranges from 10:90 to 90:10, preferably from 50:50 to 15:85. The catalyst is used in the form of pellets, which are preferably in the range of 0.1 to 1 mm. Suitably the pellets comprise crystals of zeolite-beta having a size of 200 to 2000 Ångstrom, preferably of 200 to 1000 Ångstrom. Advantageous catalyst activity and intra-pellet diffusion are thereby achieved. Crystal size may be measured by known techniques suitably transmission electron microscopy, or thickness surface area characterisation as described in B.C. Lippens and J.H. deBoer, J. Catal., 4, 319 (1965) and in J. Lynch, F. Raatz and Ch. Delalande, Studies in Surf. Science and Catalysis, Vol. 39, pp. 547-557.

Preferably the process of the invention is operated with a highly acidic zeolite-beta catalyst. The acid site density may be determined by titration for example with butylamine, or by NMR or IR techniques. A high acid site density may be beneficial in prolonging the catalyst age prior to deactivation. A highly acidic zeolite-beta catalyst may advantageously be used in the process of the invention at high olefin space velocities resulting in increased rate of production of alkylate. Although the acid strength of sites can be determined as such, it is convenient to use known techniques for comparing catalyst acid site strengths as given for example in relation to zeolite catalysts in "Introduction to Zeolite Science and Practice", van Bekkum, Flanigen, Jansen, Elsevier 1991 at pages 268 to 278.

It may be desired to increase the potential catalyst life prior to use by increasing the availability of acid sites at which reaction may take place. Suitably the catalyst may be treated to increase the acid site density by techniques known in the art. The zeolitic catalyst may be treated for example with alkali wash such as sodium hydroxide to redistribute the alumina within the catalyst, decreasing the silica-alumina lattice ratio. Alternatively, a high alumina content zeolite-beta may be synthesised for use.

By the process of the invention it is possible to operate at an acceptable selectivity, i.e. obtaining an acceptable alkylate yield for the duration of the time on stream. By this means the catalyst alkylation activity is upheld throughout partial deactivation and until full deactivation of all sites has occurred, at which point olefin breakthrough is observed. Breakthrough is conveniently indicated by gas chromatography. Hence for the duration of time on stream it would appear that oligomerisation product remains associated with the acid sites of the catalyst and is not detected in the product.

Regeneration of the catalyst may be carried out by techniques known in the art for desorbing hydrocarbonaceous deposits from acid sites, such as oxidative regeneration at elevated temperature in oxygen-rich atmosphere. The process of the invention is characterised by increased efficiency in cases in which the regeneration rate is greater than the deactivation rate. Hence techniques which increase the regeneration rate may be of particular advantage in the process.

Regeneration may be carried out continuously, for example one or more of a cascade of reactors, for example one or more beds in a multiple fixed bed downflow reactor may be operated as desired in reaction or in regeneration mode. Fresh or regenerated catalyst may be continuously added to an independent reactor or each of a cascade of reactors simultaneously with continuous catalyst withdrawal from the reactor and the withdrawn catalyst passed to a regeneration reactor.

It may be desirable to convert branched alkylate further to other products. Accordingly the process of the invention may include a feed line to a further process unit.

The process is now illustrated by way of non-limiting example.

### EXAMPLE 1

A single fixed bed reactor with one external recycle containing zeolite-beta catalyst having pellet size of 0.1 mm and a silica to alumina ratio of 12.7, was charged with liquid isobutane feedstock. The reactor and contents were heated and the reaction temperature maintained between 80 and 90C. Introduction of isobutane was maintained as liquid reactor contents were drawn off from below the bed and a part thereof externally circulated to the reactor at an extent of recycle of 100. 2-Butene was continuously added with isobutane feedstock at a paraffin to olefin ratio of 30 v/v and an olefin space velocity of 0.08 kg/kg.h. Alkylate was produced for 100 hours. Olefin conversion was greater than 99 mol% i.e. substantially complete. The results are given in Table 1 below measured over two sampling periods at 30 and 60 hours on stream. Deactivation occurred at catalyst life of 8 kg/kg.

Yield of the essentially paraffinic C5+ product was measured as 200% by weight on olefin.

**TABLE 1**

| Product obtained at sampling periods, % by weight | | |
|---|---|---|
| | 30h | 60h |
| C5/C5+ | 5 | 5 |
| C6/C5+ | 4 | 3 |
| C7/C5+ | 7 | 6 |
| C8/C5+ | 82 | 79 |
| C9+/C5+ | 2 | 7 |
| | + | + |
| | 100 | 100 |
| TMP/C8 | 78 | 72 |
| TMP is trimethylpentanes Yields are given as % of total yield in percent of C5+ or C8 as indicated. | | |

### COMPARATIVE EXAMPLE

Example 1 was repeated except that no external recycle was used. Since the catalyst deactivated very rapidly no alkylate was detected in the reactor effluent.

## Claims

1. Process for upgrading a paraffinic feedstock comprising
a) supplying the feedstock and an olefins-containing stream at a paraffin to olefin ratio greater than 2 v/v to a reactor containing a zeolite-beta catalyst and
b) removing the upgraded product characterised in that the process is operated at an olefin conversion of at least 90 mol%, and in that a part of the upgraded product is circulated in an external circulation reactor having an extent of external circulation of liquid reactor content expressed as$\frac{\text{Øvc + Øv}}{\text{Øv}} \text{of greater than 2}$ wherein Øv is the volumetric flow rate of feedstock and olefins-containing stream and Øvc is the volumetric external circulation flow rate.

2. Process according to claim 1 operated at olefin conversion of at least 95 mol%.

3. Process according to any of claims 1 and 2 characterised by an extent of external circulation as defined in claim 1 of greater than 50.

4. Process according to any of claims 1 to 3 wherein the reactor is first charged with paraffinic feedstock, an external circulation of liquid reactor contents is then fed back to the fresh feed stream and an olefins-containing stream is subsequently continuously and quantitatively charged with the paraffinic feedstock at an olefin space velocity such that conversion is maintained in excess of 90 mol%.

5. Process according to any of claims 1 to 4 characterised by an olefin space velocity in the range 0.01 to 10 kg/kg.h.

6. Process according to any of claims 1 to 5 characterised by a paraffin to olefin ratio greater than 10 v/v.

7. Process according to any of claims 1 to 6 which may be operated to a catalyst life in excess of 7 kg/kg.

8. Process according to any of claims 1 to 7 operated in one or more of a cascade of reactors in series.

9. Process according to claim 8 wherein one or more reactors are operated in catalyst regeneration mode.

10. Process according to any of claims 1 to 9 carried out at a temperature of less than 150 °C.

11. Process according to any of claims 1 to 10 wherein steady state operation within volumetric flow rate, temperature and olefin space velocity ranges as defined in the foregoing claims is achieved by use of Advanced Process Control monitoring alkylate quality together with operational process data.

12. Process according to any of claims 1 to 11 wherein the zeolite is in the form of crystals of size 200 to 2000 Ångstrom.

13. Process according to any of claims 1 to 12 wherein the catalyst is in the form of pellets of size 0.1 to 1 mm.

14. Process according to any of claims 1 to 13 wherein the catalyst has been treated to increase the acid site density.

## Patentansprüche

1. Verfahren zum Aufbessern eines paraffinischen Einsatzmaterials, umfassend:
a) Zuführen des Einsatzmaterials und eines olefinhältigen Stroms bei einem Paraffin/Olefin-Verhältnis von größer als 2 VOL./VOL. zu einem einen β-Zeolith-Katalysator enthaltenden Reaktor und
b) Abführen des aufgebesserten Produktes, dadurch gekennzeichnet, daß das Verfahren bei einer Olefinumwandlung von wenigstens 90 Mol-% ausgeführt wird und daß ein Teil des aufgebesserten Produktes in einem Reaktor mit externem Kreislauf zirkuliert wird, wobei das Ausmaß des externen Kreislaufes des Reaktor-Flüssigkeitsgehaltes, ausgedrückt als$\frac{\text{Øvc + Øv}}{\text{Øv}} \text{,}$ größer als 2 ist und worin Øv die volumetrische Strömungsgeschwindigkeit des Einsatzmaterial und Olefin enthaltenden Stroms ist und Øvc die volumetrische Strömungsgeschwindigkeit des Außenkreislaufes bedeutet.

2. Verfahren nach Anspruch 1, das bei einer Olefinumwandlung von wenigstens 95 Mol-% ausgeführt wird.

3. Verfahren nach einem der Ansprüche 1 und 2, gekennzeichnet durch ein Ausmaß an externer Zirkulation, wie in Anspruch 1 definiert, von größer als 50.

4. Verfahren nach einem der Ansprüche 1 bis 3, worin der Reaktor zuerst mit paraffinischem Einsatzmaterial beschickt wird, ein äußerer Kreislauf von flüssigem Reaktorinhalt dann dem frischen Speisestrom zugeführt wird und anschließend ein olefinhältiger Strom kontinuierlich und quantitativ mit dem paraffinischen Einsatzmaterial bei einer solchen Olefinraumgeschwindigkeit eingespeist wird, daß die Umwandlung über 90 Mol-% gehalten wird.

5. Verfahren nach einem der Ansprüche 1 bis 4, gekennzeichnet durch eine Olefinraumgeschwindigkeit im Bereich 0,01 bis 10 kg/kg.h.

6. Verfahren nach einem der Ansprüche 1 bis 5, gekennzeichnet durch ein Paraffin/Olefin-Verhältnis von größer als 10 Vol./Vol..

7. Verfahren nach einem der Ansprüche 1 bis 6, das bis zu einer Katalysatorlebensdauer von über 7 kg/kg betrieben werden kann.

8. Verfahren nach einem der Ansprüche 1 bis 7, das in einem Reaktor oder in mehreren, in Reihe angeordneten Reaktoren in Kaskade ausgeführt wird.

9. Verfahren nach Anspruch 8, worin ein oder mehrere Reaktoren im Katalysatorregenerationsmodus betrieben werden.

10. Verfahren nach einem der Ansprüche 1 bis 9, das bei einer Temperatur von unter 150°C betrieben wird.

11. Verfahren nach einem der Ansprüche 1 bis 10, worin ein Fließgleichgewichtszustand innerhalb von Volumenströmungsgeschwindigkeitsbereichen, Temperaturbereichen und Olefinraumgeschwindigkeitsbereichen, wie in den vorstehenden Ansprüchen definiert, durch Anwendung von fortgeschrittener Verfahrensregelung unter Verfolgung der Alkylatqualität zusammen mit den Verfahrensbetriebsdaten erzielt wird.

12. Verfahren nach einem der Ansprüche 1 bis 11, worin der Zeolith in Form von Kristallen mit einer Größe von 200 bis 2.000 Å vorliegt.

13. Verfahren nach einem der Ansprüche 1 bis 12, worin der Katalysator in Form von Pellets mit einer Größe von 0,1 bis 1 mm vorliegt.

14. Verfahren nach einem der Ansprüche 1 bis 13, worin der Katalysator zur Steigerung der Säurestellendichte behandelt worden ist.

## Revendications

1. Procédé de valorisation d'une charge de départ paraffinique, caractérisé en ce que :
a) on fournit la charge de départ et un courant contenant des oléfines à un rapport paraffine à oléfine supérieur à 2 v/v à un réacteur contenant un catalyseur du type zéolite-bêta et
b) on prélève le produit valorisé, où le procédé est amené à fonctionner à une conversion d'oléfine d'au moins 90% molaires dans un réacteur à circulation externe, ayant une ampleur de circulation externe du contenu du réacteur liquide exprimée par la relation$\frac{\text{Øvc + Øv}}{\text{Øv}} \text{supérieur à 2,}$ où Øv est le débit volumétrique de la charge de départ et du courant contenant les oléfines et Øvc est le débit de circulation externe volumétrique.

2. Procédé suivant la revendication 1, mis en oeuvre à une conversion d'oléfine d'au moins 95% molaires.

3. Procédé suivant l'une quelconque des revendications 1 et 2, caractérisé par une ampleur de circulation externe, telle que définie dans la revendication 1, supérieure à 50.

4. Procédé suivant l'une quelconque des revendications 1 à 3, caractérisé en ce que l'on alimente d'abord le réacteur de la charge de départ paraffinique, on réintroduit une circulation externe de contenu du réacteur liquide dans le courant d'alimentation frais et un courant contenant des oléfines est ensuite continuellement et quantitativement chargé avec la charge de départ paraffinique, à une vitesse spatiale d'oléfine telle que la conversion soit maintenue à une valeur supérieure à 90% molaires.

5. Procédé suivant l'une quelconque des revendications 1 à 4, caractérisé par une vitesse spatiale d'oléfine qui varie de 0,01 à 10 kg/kg.h.

6. Procédé suivant l'une quelconque des revendications 1 à 5, caractérisé par un rapport paraffine à oléfine supérieur à 10 v/v.

7. Procédé suivant l'une quelconque des revendications 1 à 6, qui peut être mis en oeuvre à une durée de vie du catalyseur supérieure à 7 kg/kg.

8. Procédé suivant l'une quelconque des revendications 1 à 7, mis en oeuvre dans un ou plus d'un d'une cascade de réacteurs en série.

9. Procédé suivant la revendication 8, caractérisé en ce qu'un ou plusieurs réacteurs fonctionnent selon le mode régénération de catalyseur.

10. Procédé suivant l'une quelconque des revendications 1 à 9, entrepris à une température inférieure à 150°C.

11. Procédé suivant l'une quelconque des revendications 1 à 10, caractérisé en ce que l'on parvient à un fonctionnement à état constant dans des limites de débit volumétrique, de température et de vitesse spatiale d'oléfine, telles que définies dans les revendications précédentes, par l'utilisation d'une surveillance de qualité de l'alkylat du type Advanced Process Control avec des données de traitement opérationnelles.

12. Procédé suivant l'une quelconque des revendications 1 à 11, caractérisé en ce que la zéolite se présente sous forme de cristaux d'un calibre de 200 à 2000 Ångtroms.

13. Procédé suivant l'une quelconque des revendications 1 à 12, caractérisé en ce que le catalyseur se présente sous forme de granules d'un calibre de 0,1 à 1 mm.

14. Procédé suivant l'une quelconque des revendications 1 à 13, caractérisé en ce que le catalyseur a été traité pour augmenter la densité des sites acides.
